# EUROPEAN PATENT SPECIFICATION

(11) **EP 0 788 500 B1**
(45) Date of publication and mention of the grant of the patent: **03.03.2004**
(21) Application number: 95929199.8
(22) Date of filing: 13.09.1995
(51) Int. Cl.: C07D 498/04, C07D 401/06, A61K 31/42

(54) **PROCESSES AND INTERMEDIATES FOR PREPARING 5,7-DIHYDRO-3- 2- 1-BENZYLPIPERIDIN-4-YL ETHYL]-6H-PYRROLO- 4,5-f]-1,2-BENZISOXAZOL-6-ONE**
VERFAHREN UND ZWISCHENPRODUKTE ZUR HERSTELLUNG VON 5,7-DIHYDRO-3-(2 1-BENZYLPIPERIDIN-4-YL ETHYL)-6H-PYRROLO-(4,5-F)-1,2-BENZISOXAZOL-6-ON
PROCEDES ET INTERMEDIAIRES POUR PREPARER LA 5,7-DIHYDRO-3- 2 1-BENZYLPIPERIDIN-4-YL ETHYL]-6H-PYRROLO- 4,5-f]-1,2-BENZISOXAZOL-6-ONE

(30) Priority: 26.10.1994 US 329352
(43) Date of publication of application: 13.08.1997
(73) Proprietor: PFIZER INC., New York, N.Y. 10017 (US)
(72) Inventor: DEVRIES, Keith, M./Pfizer Inc., New York, NY 10017 (US); VILLALOBOS, Anabella/Pfizer Inc., New York, NY 10017 (US)
(74) Representative: Hayles, James Richard
(86) International application number: PCT/IB1995/000755
(87) International publication number: WO 1996/013505

(56) References cited:
- WO-A-92/17475
- JOURNAL OF MEDICINAL CHEMISTRY, vol. 38, no. 15, 21 July 1995 WASHINGTON US, pages 2802-2808, A. VILLALOBOS ET AL '5,7-Dihydro-3-2-(1-phenylmethyl-4-piperid inyl)ethyl)-6H-pyrrolo(3,2-f)-1,2-benzisox azol-6-one: A potent and centrally-selective inhibitor of acetylcholinesterase with an improved margin of safety'

## Description

### Background of the Invention

The present invention relates to the compound of the formula I, below, and pharmaceutically acceptable salts and pro drugs thereof. More particularly, it relates to processes and intermediates for use in the preparation of the compound of formula I.

The compounds of formula I are cholinesterase inhibitors and are useful in enhancing memory in patients suffering from dementia and Alzheimer's disease.

The compound of formula I is disclosed in co-pending U.S. patent application number 08/127,847 (assigned to the Assignee of this application), granted as US Patents Nos 5,538,984 and 5,750,542.

The compound of formula I, and a method for its, production, are also disclosed in WO 92/17475.

Alzheimer's disease is associated with degeneration of cholinergic neurons in the basal forebrain that play a fundamental role in cognitive functions, including memory. Becker et al., Drug Development Research, 12, 163-195 (1988). As a result of such degeneration, patients suffering from the disease exhibit a marked reduction in acetylcholine synthesis, choline acetyltransferase activity, acetylcholinesterase activity and choline uptake.

It is known that acetylcholinesterase inhibitors are effective in enhancing cholinergic activity and useful in improving the memory of Alzheimer's patients. By inhibiting the acetylcholinesterase enzyme, these compounds increase the level of the neurotransmitter acetylcholine in the brain and thus enhance memory. Becker et al., supra, report that behavioral changes following cholinesterase inhibition appear to coincide with predicted peak levels of acetylcholine in the brain. They also discuss the efficacy of the three known acetylcholinesterase inhibitors physostigmine, metrifonate, and tetrahydroaminoacridine.

### Summary of the Invention

The present invention relates to processes and intermediates, and processes for the preparation of said intermediates, for the preparation of the compound of the formula I below.

Thus, according to the present invention, there is provided a process for preparing the compound having the formula 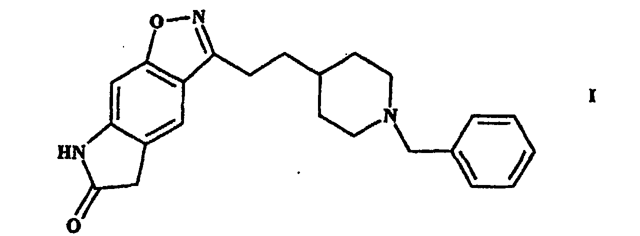 which comprises:
i) heating a compound of formula 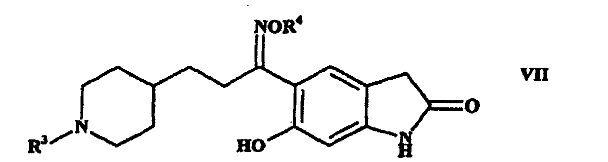 wherein R³ is R⁴ or benzyl and R⁴ is selected from R⁵-C(=O)-, R⁵SO₂- and R⁵OC(=O)- and R⁵ is (C₁-C₆)alkyl, (C₆-C₁₀)aryl or aryl(C₁-C₆)alkyl;
   under one of the following conditions:
   1. at the reflux temperature of a mixture formed with an appropriate base such as pyridine or 2,6-lutidine;
   2. at a temperature of about 130°C in a polar solvent in the presence of a base; or
   3. at the reflux temperature of a mixture formed with THF and 2,6-lutidine as base;
   with the proviso that when R³ in the resultant product is R⁴, said product is
ii) further treated with an aqueous mineral acid at a temperature in the range of from about room temperature to about reflux, followed by
iii) treatment of the product of ii) with
   a) a benzylating agent in the presence of a base, or
   b) benzaldehyde in the presence of a reducing agent and an acid.

Preferably, in step ii) above, said acid is 3-12N HCl. More preferably, said acid is 6N HCl and said temperature is from about 80 to about 90°C.

Preferably, said benzylating agent is a benzyl halide, for example benzyl bromide.

Preferably, the base of step iii) a) above is triethanolamine.

Preferably, the base of step i) above is 2,6-lutidine.

Preferably, in step iii) b) above, said reducing agent is triacetoxyborohydride and said acid is acetic acid.

More particularly, the invention relates to a process for the preparation of the compound of formula I which comprises
a) treating a compound of the formula 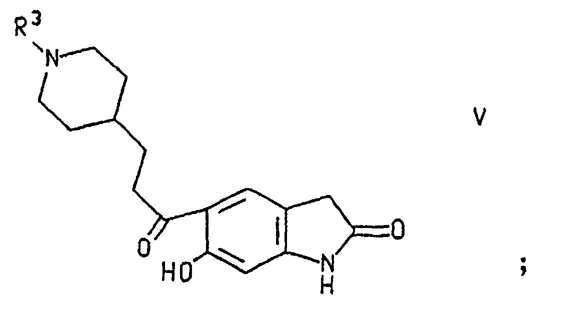 wherein R³ is as defined above;
   with a hydroxylamine salt in the presence of an appropriate base to form the compound of formula 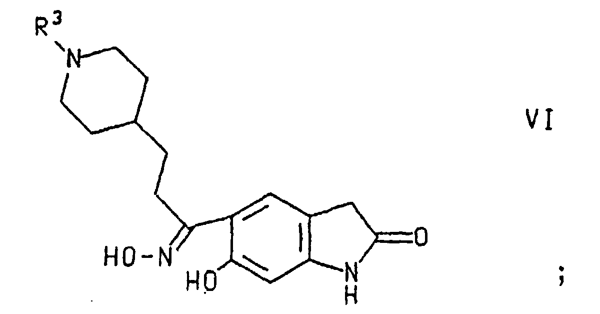 wherein R³ is as defined above;
b) treating the compound of formula VI with an acylating agent selected from the group comprising acyl or aroyl anhydrides, halides and esters; a carbamylating agent; or a sulfonating agent selected from the group comprising alkyl, aryl or aralkyl sulfonyl halides and anhydrides, and halides of groups of the formula R⁵OC(=O)-, in the presence of a base, to form the compound of formula 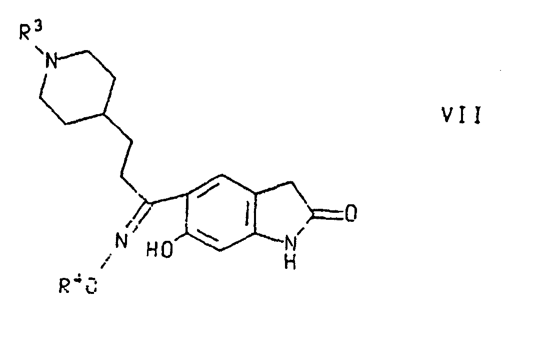 wherein R³ and R⁴ are as defined above;
c) treating the compound of formula VII with a base at an elevated temperature, in accordance with step i) above, to form
   i) when R³ is 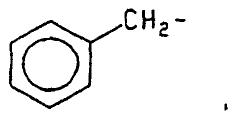 the compound of formula I; or
   ii) when R³ is R⁴, the compound of the formula 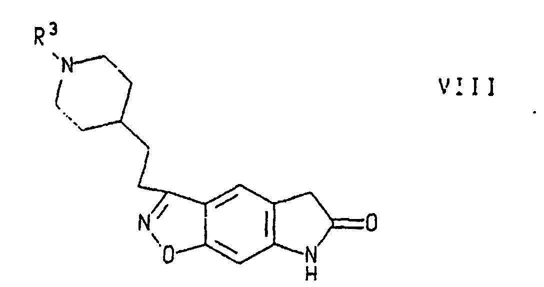
wherein R³ is as defined above;
2) treating the compound of formula VIII above with an aqueous mineral acid at an elevated temperature, in accordance with step ii) above, and then with a base to form the compound of formula 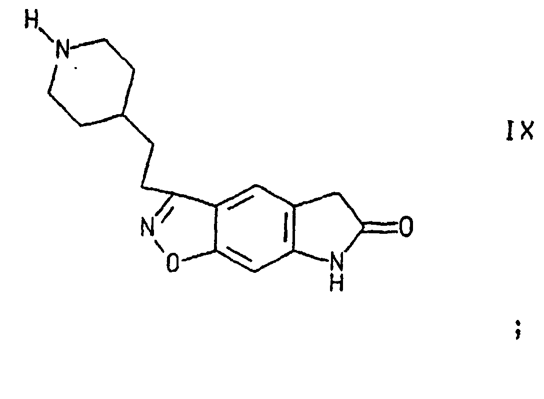 and
3) a) treating the compound of formula IX with a benzylating agent in the presence of a base; or b) benzaldehyde in the presence of a reducing agent and an acid, to form the compound of formula I.

This invention also relates to compounds of the formula 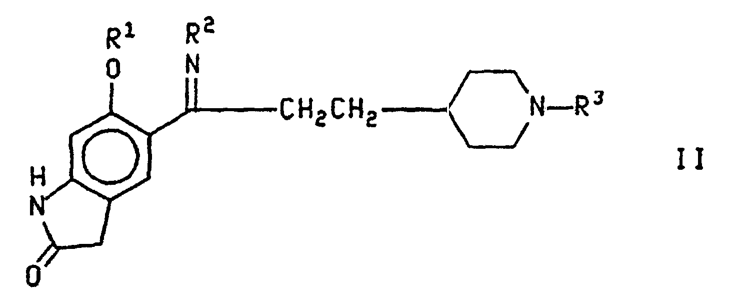 wherein:
R¹ is H and R² is OH or OR⁴ wherein R⁴ is R⁵C(=O), R⁵OC(=O) or R⁵SO₂ wherein R⁵ is (C₁-C₆)alkyl, (C₆-C₁₀)aryl or (C₆-C₁₀)aryl(C₁-C₆)alkyl; and R³ is R⁴ or benzyl. These compounds are useful as intermediates in the synthesis of compounds of the formula I.

More specifically, this invention relates to compounds of the formulae 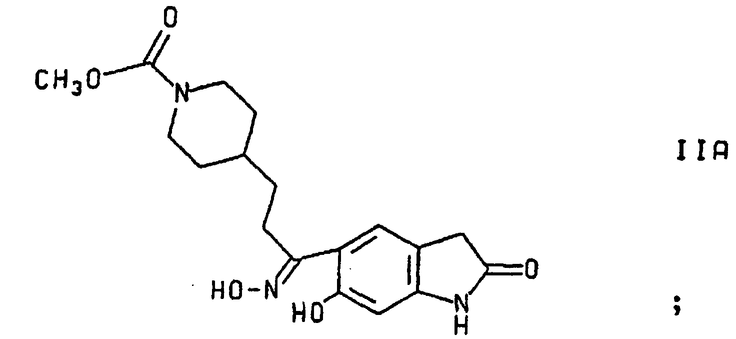 and

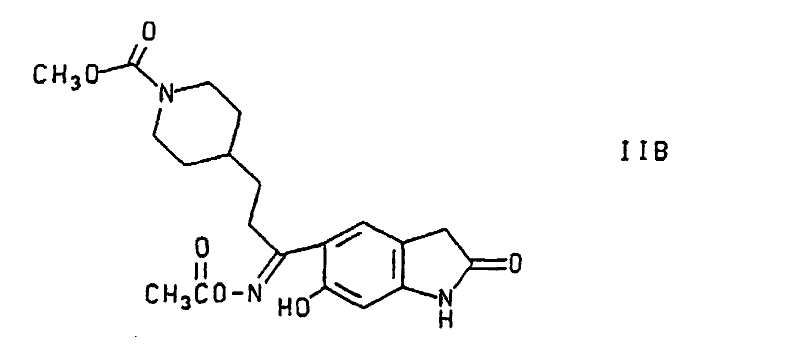

### Detailed Description of the Invention

The preparation of the compound of formula t, and certain of the starting materials used therein is illustrated in the following reaction schemes. Except where otherwise stated, in the reaction schemes and discussion that follow, R¹, R², R³, R⁴, R⁶, R⁷ and R⁸ and structures I, II, IIA and IIB are defined as above.

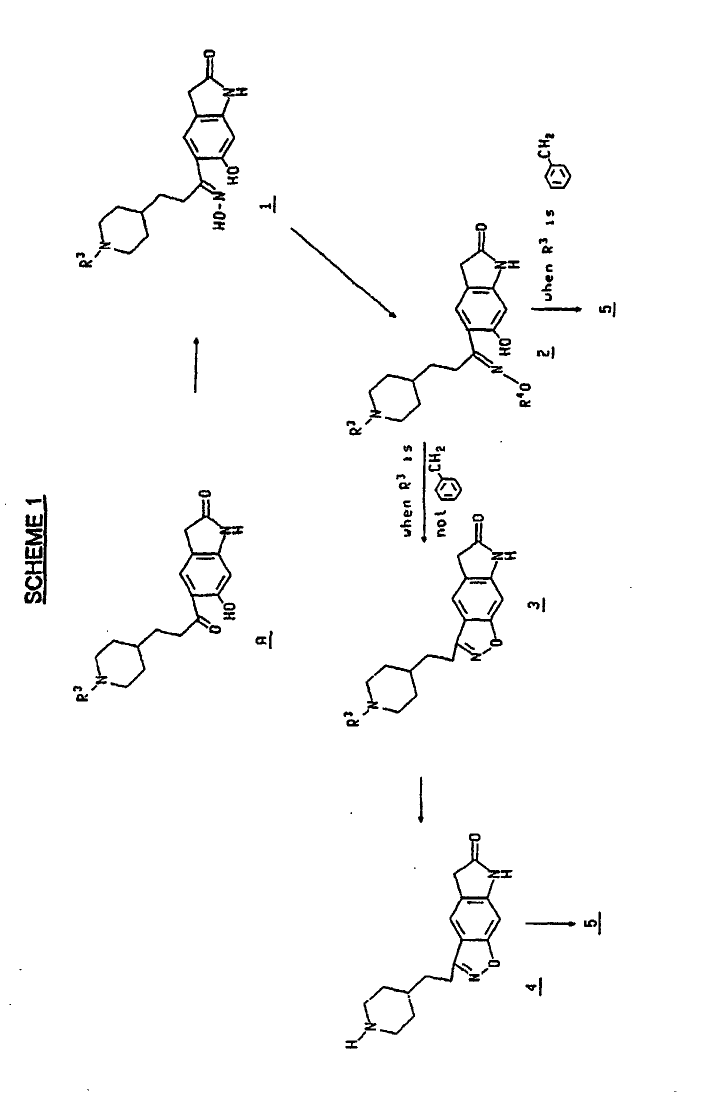

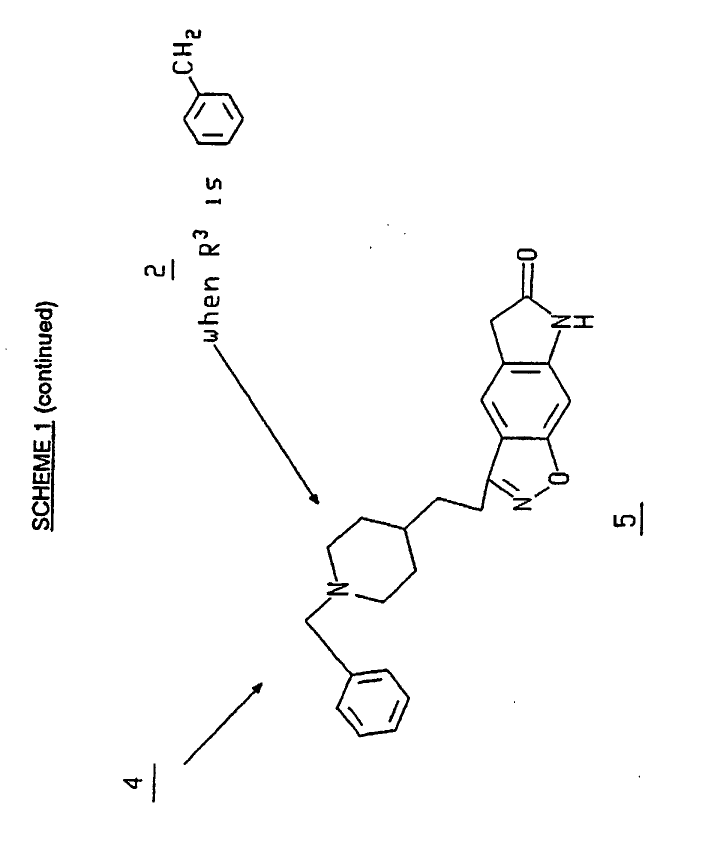

Compound A is converted to compound 1 by reaction with an hydroxylamine salt, preferably the hydrochloride, in the presence of an appropriate base such as potassium or sodium hydroxide, sodium acetate or pyridine, preferably aqueous potassium hydroxide or aqueous sodium acetate, in a polar solvent such as methanol, ethanol, water or mixtures thereof, preferably a mixture of ethanol and water, at a temperature from about room ternperature to about 120°C, preferably about 80°C. The starting material A can be prepared by methods known in the art.

Compound 1 is then converted to compound 2 by treatment with an appropriate acylating or sulfonating agent, such as an anhydride, halide or ester, or a carbamyl halide in a polar solvent, such as those described above, or ethers such as tetrahydrofuran (THF). Temperatures for this reaction may range from about room temperature to the reflux temperature of the solvent. Preferably the reaction is carried out in THF at about room temperature.

Compound 3 may be prepared by heating neat compound 2 at a temperature from about 125°C to about 200°C under atmospheric pressure or reduced pressure (e.g., from about 0.01 mm Hg (1.33 X 10⁻⁵ bars) to about 760 mm Hg(1.01 bars)). Ring closure is preferably accomplished by heating the compound 2 at reflux in an appropriate base such as pyridine or 2,6-lutidine or by heating at a temperature of about 130°C in a polar solvent such as dimethylformamide (DMF), dimethylsulfoxide (DMSO) or THF in the presence of several equivalents of an appropriate base such as pyridine or 2,6-lutidine. Preferably the reaction is carried out in refluxing THF and the base is 2,6-lutidine.

Alternatively, compound 3 may be prepared from compound 1 directly by reaction of compound 1 with an acyl or suifonyl chloride such as oxalyl or thionyl chloride in the presence of an aromatic amine such as pyridine (See Kalkote et al., Aust. J. Chem. 1977, 30, 1847). Suitable solvents include polar solvents such as diethyl ether or THF. Temperatures can range from about 0°C to about room temperature. Another method of closure involves treatment of compound 1, with one or less equivalents of a base such as potassium hydroxide in a polar solvent such as methanol at temperatures ranging from about room temperature to about 100°C (Crabbe et al., J. Chem. Soc. Perkin Trans. I, 1973, 2220).

When R³ is C₆H₅CH₂- compound 3 is the same as compound 5.

When R³ is a nitrogen protecting group it can be removed from compound 3 to form compound 4 by methods known to those skilled in the art. For example, when R³ is (CH₃)₃CO(C=O)-(BOC) or another carbamate, it can be removed with an acid such as hydrogen bromide (gas or aqueous), hydrogen chloride (gas or aqueous) or trifluoroacetic acid. in the case of trifluoroacetic acid, a t-butyl cation scavenger such as thioanisole may be added. When an acid is used as the deprotecting agent an acid addition salt of compound 4 is produced rather than the free base of such compound 4. Appropriate solvents include non-polar solvents such as methylene chloride, as well as polar solvents such as diethyl ether, ethyl acetate, dioxane, alcohols (e.g. methanol or ethanol) and water. Temperatures may range from about -20°C to about the reflux temperature of the solvent. Where the protecting group is an acyl or carbamate group the preferred deprotecting composition is 3-12N, preferably 6 N, HCl (aqueous) and the temperature is from about room temperature to about reflux, preferably from about 80 to about 90°C.

Alternatively, when R³ is BOC, it can be removed with a trialkylsilyltrifluoromethanesulfonate derivative such as trimethylsilyl-, triethylsilyl-, or t-butyldimethylsilyl-trifluoromethanesulfonate in the presence of an aromatic or tertiary amine base such as 2,6-lutidine or triethylamine. Appropriate solvents for this reaction include nonpolar solvents such as methylene chloride and polar aprotic solvents such as THF, diethyl ether or DMF. Temperatures may range from about -20°C to room temperature. It is preferable to use trimethylsilyltrifluoromethane-sulfonate and 2,6-lutidine in methylene chloride at a temperature from about 0°C to about room temperature.

Compound 4 is preferably converted to compound 5 by treatment with a benzylating agent, such as a benzyl halide, in the presence of a base. A preferred benzylating agent is benzyl bromide and a preferred base is triethanolamine. Solvents for use in this reaction include chlorinated hydrocarbons, as described above, in (C₁-C₆)alkanoic acids, (C₁-C₆)alkanols, ethers, such as diethylether, cyclic ethers, such as tetrahydrofuran (THF) and dioxane and mixtures thereof. The preferred solvent is THF.

Compound 4 may also be converted to compound 5 by reductive amination with benzaldehyde, in an inert solvent, as described above in the presence of a reducing agent.

Useful reducing agents include borohydrides, such as those of the alkali metals, NaHB(CN)₃ and triacetoxyborohydride; and borane complexes such as those with pyridine and triethylamine. A preferred solvent is CH₂Cl₂/acetic acid and a preferred reducing agent is triacetoxyborohydride.

In each of the above reactions, unless otherwise indicated, pressure is not critical. Pressures in the range of about 0.5-3 atm (0.5-3 bars) are suitable, and ambient pressure (generally, about one atmosphere) is preferred as a matter of convenience. Also, for those reactions where the preferred temperature varies with the particular compounds reacted, no preferred temperature is stated. For such reactions, preferred temperatures for particular reactants may be determined by monitoring the reaction using thin layer chromatography.

The compounds of formula I and their pharmaceutically acceptable acid addition salts (hereinafter referred to as the "active compounds") may be administered to a patient by various methods, for example, orally as capsules or tablets, parentally as a patch, sterile solution or suspension, and in some cases, intravenously in the form of a solution. The free base compounds of the invention may be formulated and administered in the form of their pharmaceutically acceptable acid addition salts.

The daily dose of the active compounds is generally in the range of from about 0.005 to about 300 mg/day, optionally from about 0.01 to 300 mg/day, and preferably from about 0.01 to about 30 mg/day for the average adult human, and may be administered in single or divided doses.

When incorporated into a solution or suspension, for parenteral administration, the active compounds are present in a concentration of at least about 0.0005 weight percent, and preferably between about 0.001 to about 30 weight percent (based on the total weight of the unit). The parenteral dosage unit typically contains between about 0.5 to about 100 mg of active compound(s).

The active compounds may be administered orally with an inert diluent or an edible carrier, or they may be enclosed in gelatin capsules or compressed into tablets. Such preparations should contain at least about 0.0005% of active compound(s), but the concentration may vary depending upon the particular form and may be from about 0.001 to about 30 weight percent (based on the total weight of the unit). The oral dosage unit typically contains between about 0.01 mg to about 30 mg of active compound.

The cholinesterase inhibiting activity of the active compounds may be determined by a number of standard biological or pharmacological tests. One such procedure for determining cholinesterase inhibition is described by Ellman et al. in "A New and Rapid Colorimetric Determination of Acetylcholinesterase Activity", Biochem. Pharm. 1, 88, (1961).

The present invention is illustrated by the following examples. It will be understood, however, that the invention is not limited to the specific details of these examples. Melting points are uncorrected. Proton nuclear magnetic resonance spectra (¹H NMR) were measured for solutions in deuterochloroform (CDCl₃) except where otherwise noted and peak positions are expressed in parts per million (ppm). The peak shapes are denoted as follows: s, singlet; d, doublet; t, triplet; q, quartet; m, multiplet; br, broad. Frequencies (J) are expressed in Hertz.

### Example 1

### Preparation of 5-(3-[N-(Methoxycarbonyl)piperidin-4-yl]proprionyl)-1,3-dihydro-6-hydroxy-2H-indol-2-one

a) Preparation of 3-pyridin-4-ylpropen-2-oic acid
   To a solution of pyridin-4-ylcarboxaldehyde (100 g, 0.93 mol) in 100 mL of pyridine (1.0 equiv.) was added malonic acid (100 g, 0.96 mol) at 90°C. After CO₂ evolution subsided, the reaction slurry was diluted with methanol. The title compound was isolated as a white solid by filtration.
   ¹H NMR (CH₃CO₂H-d₄) δ 11.70 (s, 1H), 8.85 (d, 2H), 7.95 (d, 2H), 7.80 (d, 1H), 6.90 (d, 1H).
b) Preparation of 3-piperidin-4-ylpropanoic acid
   The product of step a) was dissolved in 150 mL of 2 N HCl and treated with 10 weight % of 5% Rh-C and hydrogen until hydrogen gas uptake ceased. The catalyst was removed by filtration and the resulting solution used directly in the next step.
   ¹H NMR (D₂O) δ 3.25 (m, 2H), 2.80 (m, 2H), 225 (t, 2H), 1.75 (m, 2H), 1.50-1.10 (m, 5H). FABMS (M+1)⁺ = 157
c) Preparation of 3-[N-(methoxycarbonyl)-piperidin-4-yl]proprionic acid
   The solution from step b) was brought to pH 12 with aqueous potassium hydroxide. To this solution was added 21 mL methyl chloroformate (0.27 mol). After the reaction was complete, the solution was brought to pH 1 with 6 N HCl and extracted with dichloromethane. The organic layer was dried with sodium sulfate and the dichloromethane displaced with isopropyl ether. The product was isolated as a solid by filtration. Yield 39 gm, 84%. M.p. 89-90°C.
   ¹H NMR (CDCl₃) δ 4.10 (m, 2H), 3.65 (s, 3H), 2.70 (m, 2H), 2.35 (t, 2H), 1.80-1.10 (m, 7H).
d) Preparation of 3-[N-(methoxycarbonyl)-piperidin-4-yl]proprionyl chloride
   To a solution of 49.5 gm (0.23 mol) of the compound of step c), in dichloromethane, was added 0.25 mL of dimethylformamide and 21 mL of oxalyl chioride (0.25 mol). After gas evolution subsided, formation of the title compound was complete. The resulting solution was used directly into the next step.
e) The solution from step d), comprising 1.5 equiv of the title compound thereof, was added to a slurry of 25 gm (0.15 mol) of 6-methoxyindol-2-one and 102 gm (0.77 mol) of aluminum trichloride in 625 mL dichloromethane. After 2 hours the reaction was complete. The reaction was quenched with water and the product partitioned between pH 1 aqueous HCl and dichloromethane. The organic layer was separated, dried with sodium sulfate, and the dichloromethane displaced with isopropyl ether. The title compound of this Example was isolated as a solid by filtration. Yield 49 gm, 94%. M.p. 199-200°C.
   ¹H NMR (CDCl₃) δ 13.10 (s, 1H), 8.80 (s, 1H), 7.55 (s, 1H), 6.50 (s, 1H), 4.15 (m, 2H), 3.70 (s, 3H) 3.50 (s, 2H), 2.95 (t, 2H) 2.75 (m, 2H), 1.85-1.15 (m, 7H).
   FABMS (M+1)⁺ = 346.

### Example 2

### Preparation of 5-(3-[N-(Methoxycarbonyl)piperidin-4-yl]proprionyl)-1,3-dihydro-6-hydroxy-2H-indol-2-one, 5-oxime

A slurry of 6.0 gm (17 mmol) of the title compound of Example 1 in 4:1 ethanol/water was treated with 3.6 gm (52 mmol) of hydroxylamine hydrochloride and sodium acetate (4.3 gm, 52 mmol). The slurry was stirred at 80°C overnight, at which time the reaction was complete. The ethanol was removed in vacuo and the title compound was isolated as a solid by filtration. Yield 5.3 gm, 86%. M.p. 247-248°C.
¹H NMR (CDCl₃ + DMSO-d₆) δ 12.10 (s, 1H), 10.95 (s, 1H), 10.30 (s, 1H), 7.60 (s, 1H), 6.30 9s, 1h), 4.00 (m, 2H), 3.60 (s, 2H), 2.80-2.60 (m, 4H), 1.80-1.00 (m, 7H).
FABMS (M+1)⁺ = 361

### Example 3

### Preparation of 5-(3-[N-(Methoxycarbonyl)piperidin-4-yl]proprionyl)-1,3-dihydro-6-hydroxy-2H-indol-2-one, 5-oxime acetate

A slurry of 5.2 gm (14 mmol) of the title compound of Example 2 in 100 mL tetrahydrofuran was treated with 1.2 gm (14 mmol) sodium acetate and 1.4 mL (15 mmol) acetic anhydride and stirred overnight at room temperature. The title compound was isolated from the crude reaction mixture, as a solid, by filtration. Yield 5.3 gm, 92%. M.p. = 139-140°C.
¹H NMR (CDCl₃) δ 11.80 (s, 1H), 8.15 (s, 1H), 7.20 (s, 1H), 6.55 (s, 1H), 4.20 (m, 2H), 3.70 (s, 3H (s, 2H), 2.95-270 (m, 4H), 2.225 (s, 3H), 1.95-1.10 (m, 7H).
FABMS (M+Na)⁺ = 426

### Example 4

### Preparation of 5,7-Dihydro-3-(2-[N-(methoxycarbonyl)-piperidin-4-yl]ethyl)-6H-pyrrolo[4,5-f]-1,2-benzisoxazol-6-one

A slurry of 3.6 gm (8.8 mmol) of the title compound of Example 3 in 50 mL tetrahydrofuran containing 3.1 mL (26 mmol) 2,6-lutidine was heated at 65°C overnight. The solution was then cooled to 0°C and the title compound precipitated upon addition of isopropyl ether. The title compound was isolated by filtration. Yield 2.2 gm. 72%.
¹H NMR (CDCl₃ *δ* 8.70 (s, 1H), 7.45 (s, 1H), 7.05 (s, 1H), 4.15 (m. 2H). 3.70 (s, 3H), 3.60 (s, 2H), 3.00 (t, 2H), 2.75 (m, 2H), 1.85-1.05 (m, 7H).
FABMS (M+1)⁺ = 343

### Example 5

### Preparation of 5,7-Dihydro-3-[2-[1-benzylpiperidin-4-yl]ethyl]-6H-pyrrolo[4,5-f]-1,2-benzisoxazol-6-one

a) A slurry of 0.65 gm (1.9 mmol) of the title compound of Example 4 in 6 N HCl was heated at 100°C overnight. The solution was then cooled to 0°C and the product precipitated upon basification to pH 10 with aqueous NaOH. The product was isolated as a solid by filtration. Yield 420 mg, 78%. M.p. 248-9°C
   ¹H NMR (CDCl₃) + CD₃OD) δ 7.70 (s, 1H), 7.00 (s, 1H), 3.60 (s, 2H), 3.05 (m, 2H), 2.95 (t, 3H), 2.55 (m, 2H), 1.85-.10 (m, 7H).
   FABMS (M+1)⁺ = 285
b)
   i) A slurry of 1.44 gm of the product of step a) (5.1 mmol) in 50 mL of THF was treated with 0.61 mL of benzyl bromide (5.1 mmol) and 0.76 gm of triethanolamine (5.1 mmol). The reaction mixture was heated overnight at 65°C and then filtered. The filtrate was evaporated and the resultant solid recrystallized from 20 mL of hot ethyl acetate. Upon cooling, the title compound was isolated as a solid by filtration (1.40 gm, 74%). M.p. 118-119°C.
      ¹H NMR (CDCl₃) δ 9.05 (s, 1H), 7.45 (s, 1H), 7.20-7.40 (m, 5H), 7.00 (s, 1H), 3.65 (s, 2H), 3.60 (s, 2H),3.00-2.85 (m, 4H), 2.05-1.25 (m, 9H).
      FABMS (M+1)⁺ = 375.
   ii) A slurry of 1.44 gm (5.1 mmol) of the product of step a), in dichloroethane, was treated with benzaldehyde (1.5 equiv.), acetic acid (1.0 equiv.), and triacetoxyborohydride (3.0 equiv.) and stirred overnight at room temperature. The reaction was quenched by addition of aqueous sodium bicarbonate and then diluted with dichloromethane. The organic layer was separated, dried with sodium sulfate, and concentrated in vacuo. The crude solid was recrystallized from ethyl acetate to provide the title compound.

## Claims

1. A process for preparing the compound having the formula 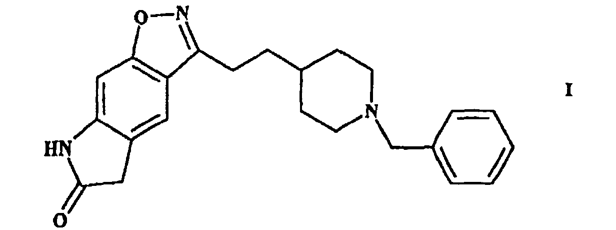 which comprises:
i) heating a compound of formula 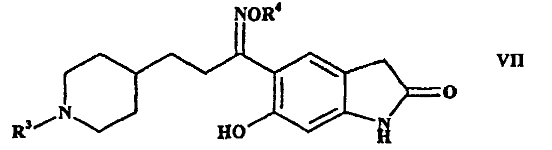 wherein R³ is R⁴ or benzyl and R⁴ is selected from R⁵-C(=O)-, R⁵SO₂- and R⁵OC(=O)- and R⁵ is (C₁-C₆)alkyl, (C₆-C₁₀)aryl or aryl(C₁-C₆)alkyl;
under one of the following conditions:
1. at the reflux temperature of a mixture formed with an appropriate base such as pyridine or 2,6-lutidine;
2. at a temperature of about 130°C in a polar solvent in the presence of a base; or
3. at the reflux temperature of a mixture formed with THF and 2,6-lutidine as base;
with the proviso that when R³ in the resultant product is R⁴, said product is
ii) further treated with an aqueous mineral acid at a temperature in the range of from about room temperature to about reflux, followed by
iii) treatment of the product of ii) with
a) a benzylating agent in the presence of a base, or
b) benzaldehyde in the presence of a reducing agent and an acid.

2. The process of claim 1 wherein the acid of step ii) is 3-12N HCl.

3. The process of claim 2 wherein said acid is 6N HCl and said temperature is from about 80 to about 90°C.

4. The process of claim 1 wherein said benzylating agent is a benzyl halide.

5. The process of claim 4 wherein said benzyl halide is benzyl bromide.

6. The process of claim 1 wherein the base of step iii) a) is triethanolamine.

7. The process of claim 1 wherein the base of step i) is 2,6-lutidine.

8. The process of claim 1 step iii) b) wherein said reducing agent is triacetoxyborohydride and said acid is acetic acid.

9. The process of claim 1 wherein the compound of formula VII is prepared by treating the compound of formula 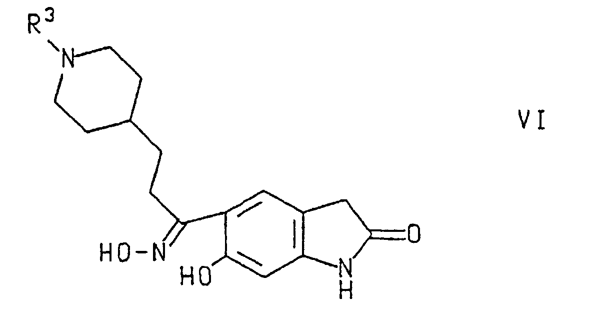 wherein R³ is as defined above;
with an acylating, carbamylating or sulfonating agent in the presence of a base.

10. The process of claim 9 wherein said acylating agent is acetic anhydride and said base is sodium acetate.

11. The process of claim 9 wherein the compound of formula VI is prepared by treating the compound of formula 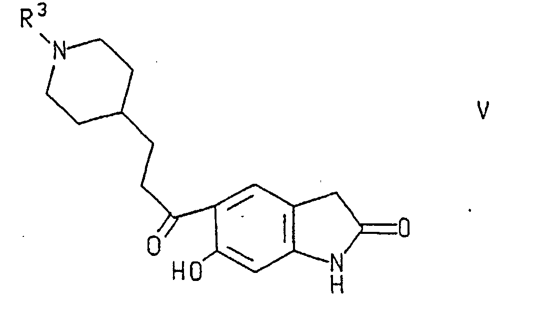 wherein R³ is as defined above, with hydroxylamine salt in the presence of a weak base.

12. The process of claim 11 wherein said salt is the hydrochloride and said weak base is sodium acetate.

13. A process for preparing a compound having the formula 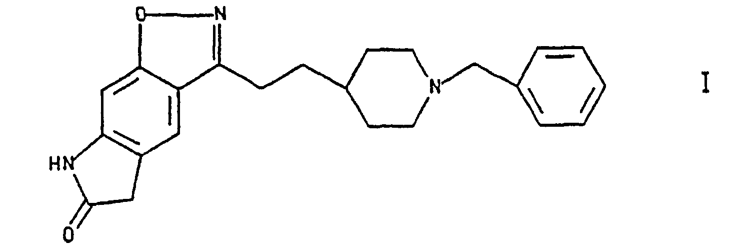 which comprises the steps of
a) treating a compound of the formula 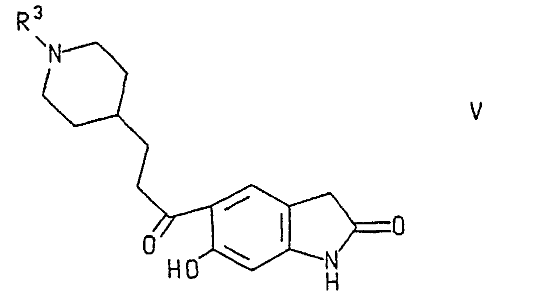 wherein R³ is as defined above;
with a hydroxylamine salt in the presence of a weak base to form the compound of formula 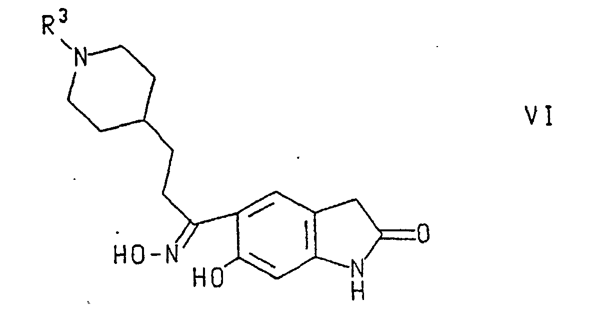 wherein R³ is as defined above;
b) treating the compound of formula VI with an acylating, carbamylating or sulfonating agent in the presence of a base to form the compound of the formula 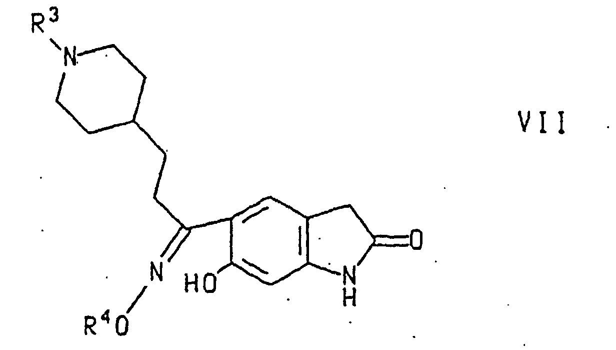 wherein R³ and R⁴ are as defined above; and
c) treating the compound of formula VII with a base at an elevated temperature, in accordance with step i) of claim 1 above, to form
i) when R³ is 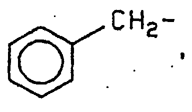 the compound of formula I; or
ii) when R³ is R⁴ a product of the formula 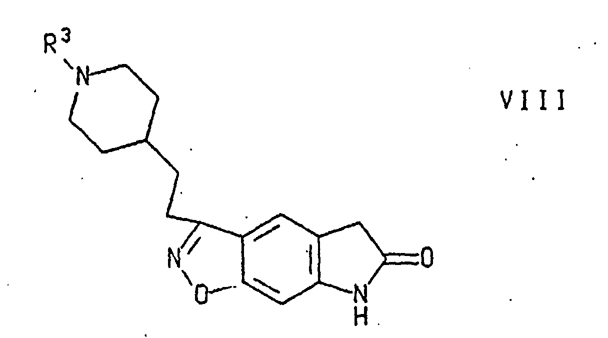 wherein R³ is as defined above;
iii) treating the compound of formula VIII with an aqueous mineral acid at an elevated temperature, in accordance with step ii) of claim 1 above, and then with a base to form the compound of formula 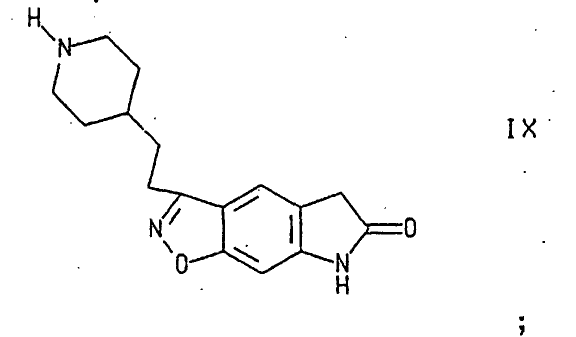
and iv) treating the compound of formula IX with a) a benzylating agent in the presence of a base or b) benzaldehyde in the presence of a reducing agent and an acid to form the compound of formula I.

14. A compound of the formula 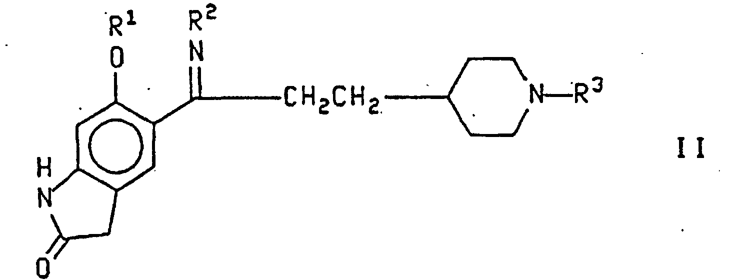 wherein
R¹ is H and R² is OH or OR⁴ wherein R⁴ is R⁵C(=O), R⁵OC(=O) or R⁵SO₂ wherein R⁵ is (C₁-C₆)alkyl, (C₆-C₁₀)aryl or (C₆-C₁₀)aryl(C₁-C₆)alkyl; and R³ is R⁴ or benzyl.

15. The compound of claim 14 wherein R¹ is hydrogen and R² is hydroxyl or OR⁴ and R³ and R⁴ are as defined above.

16. The compound of claim 15 wherein R² is OH.

17. The compound of claim 15 wherein R² is OR⁴ and R⁴ is as defined above.

18. The compound of claim 14 wherein R³ is R⁴ and R⁴ is as defined above.

19. The process of claim 13 step c) iv) wherein said benzylating agent is benzyl bromide and said base is triethanolamine.

20. The process of claim 13 step b) wherein said base is 2,6-lutidine.

## Patentansprüche

1. Verfahren zur Herstellung der Verbindung der Formel 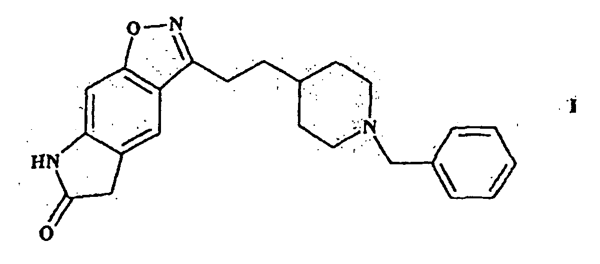 das umfasst:
i) dass eine Verbindung der Formel 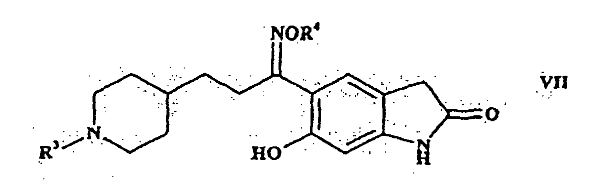 worin R³ R⁴ oder Benzyl ist und R⁴ ausgewählt ist aus R⁵-C(=O)-, R⁵SO₂- und R⁵OC(=O)- und R⁵ (C₁-C₆)-Alkyl, (C₆-C₁₀)-Aryl oder Aryl-(C₁-C₆)-alkyl ist;
unter einer der folgenden Bedingungen:
1. bei Rückflusstemperatur einer Mischung, die mit einer geeigneten Base, wie Pyridin oder 2,6-Lutidin gebildet wird;
2. bei einer Temperatur von etwa 130°C in einem polaren Lösungsmittel in Gegenwart einer Base oder
3. bei Rückflusstemperatur einer Mischung, die mit THF und 2,6-Lutidin als Base gebildet wird,
unter dem Vorbehalt, dass dann, wenn R³ in dem entstehenden Produkt R⁴ ist, das Produkt
ii) weiter mit einer wässrigen Mineralsäure bei einer Temperatur im Bereich von etwa Raumtemperatur bis etwa Rückflusstemperatur behandelt wird und anschließend
iii) das Produkt von ii) mit
a) einem Benzylierungsmittel in Gegenwart einer Base oder
b) Benzaldehyd in Gegenwart eines Reduktionsmittels und einer Säure behandelt wird.

2. Verfahren nach Anspruch 1, wobei die Säure von Stufe ii) 3 bis 12 n HCl ist.

3. Verfahren nach Anspruch 2, wobei die Säure 6 n HCl ist und die Temperatur etwa 80 bis etwa 90°C ist.

4. Verfahren nach Anspruch 1, wobei das Benzylierungsmittel Benzylhalogenid ist.

5. Verfahren nach Anspruch 4, wobei das Benzylhalogenid Benzylbromid ist.

6. Verfahren nach Anspruch 1, wobei die Base in Stufe iii) a) Triethanolamin ist.

7. Verfahren nach Anspruch 1, wobei die Base von Stufe i) 2,6-Lutidin ist.

8. Verfahren nach Anspruch 1 Stufe iii) b), wobei das Reduktionsmittel Triacetoxyborhydrid ist und die Säure Essigsäure ist.

9. Verfahren nach Anspruch 1, wobei die Verbindung der Formel VII hergestellt wird, indem die Verbindung der Formel 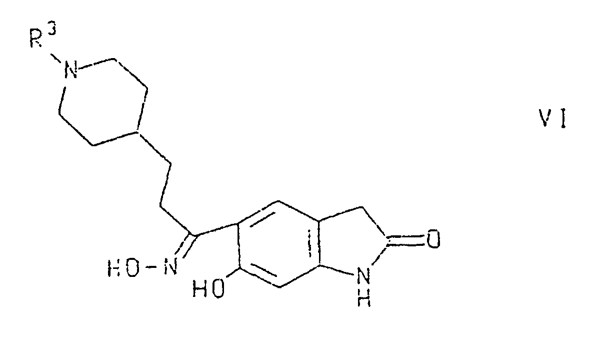 worin R³ wie oben definiert ist, mit einem acylierenden, carbamylierenden oder sulfonierenden Mittel in Gegenwart einer Base behandelt wird.

10. Verfahren nach Anspruch 9, wobei das Acylierungsmittel Essigsäureanhydrid ist und die Base Natriumacetat ist.

11. Verfahren nach Anspruch 9, wobei die Verbindung der Formel VI hergestellt wird, indem die Verbindung der Formel 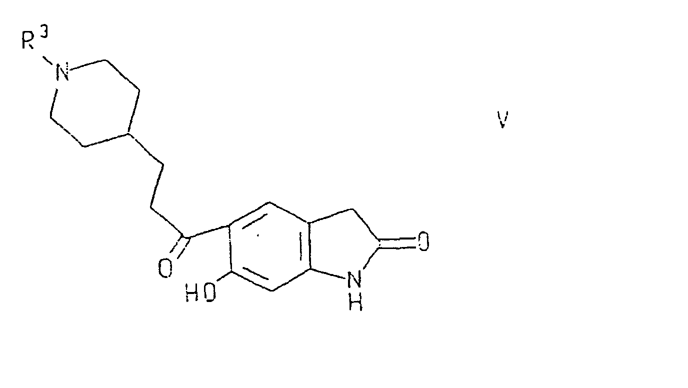 worin R³ wie oben definiert ist, mit Hydroxylaminsalz in Gegenwart einer schwachen Base behandelt wird.

12. Verfahren nach Anspruch 11, wobei das Salz das Hydrochlorid ist und die schwache Base Natriumacetat ist.

13. Verfahren zur Herstellung einer Verbindung der Formel 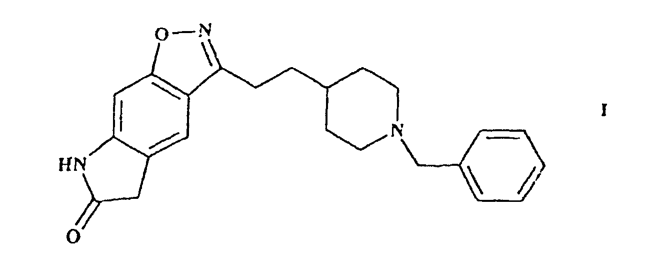 das die Stufen umfasst, dass
a) eine Verbindung der Formel 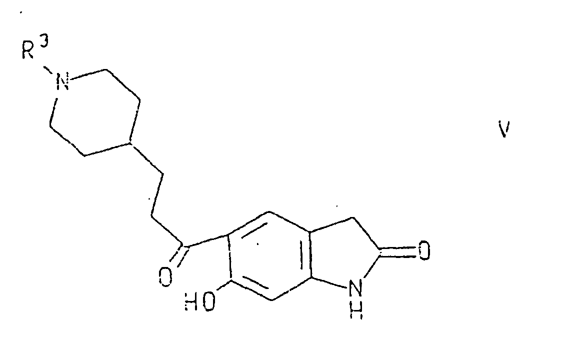 worin R³ wie oben definiert ist, mit einem Hydroxylaminsalz in Gegenwart einer schwachen Base behandelt wird, um die Verbindung der Formel 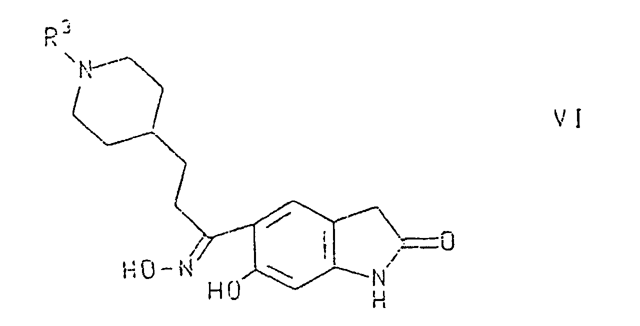 worin R³ wie oben definiert ist, zu bilden;
b) die Verbindung der Formel VI mit einem acylierenden, carbamylierenden oder sulfonierenden Mittel in Gegenwart einer Base behandelt wird, um die Verbindung der Formel 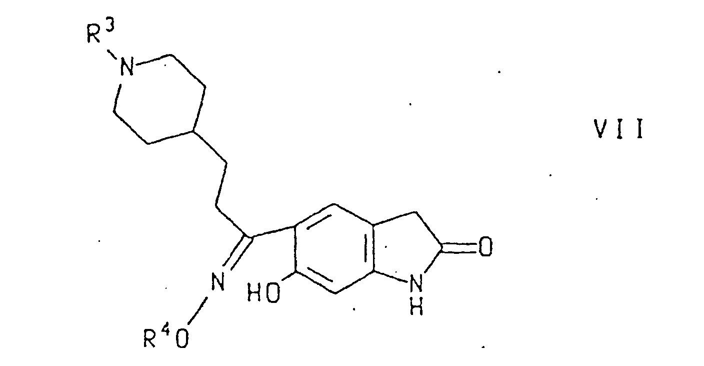 worin R³ und R⁴ wie oben definiert sind, zu bilden und
c) die Verbindung der Formel VII mit einer Base bei erhöhter Temperatur gemäß Stufe i) von Anspruch 1 behandelt wird, um
i) wenn 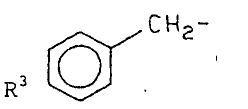 ist, die Verbindung der Formel I zu bilden oder
ii) wenn R³ R⁴ ist, ein Produkt der Formel 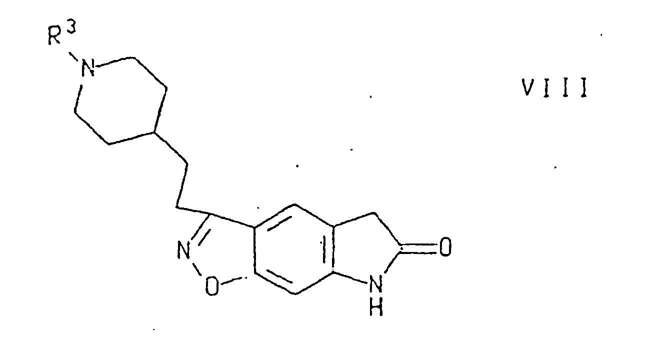 worin R³ wie oben definiert ist, zu bilden;
iii) die Verbindung der Formel VIII mit einer wässrigen Mineralsäure bei erhöhter Temperatur gemäß Stufe ii) von Anspruch 1 und dann mit einer Base behandelt wird, um die Verbindung der Formel 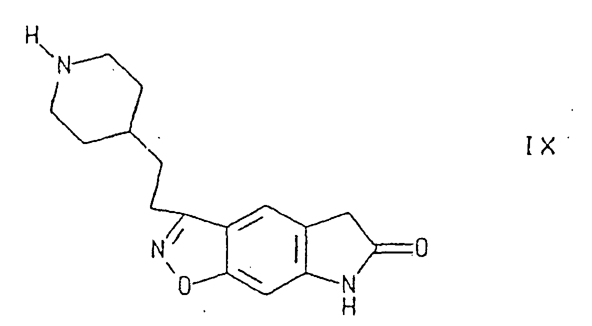 zu bilden und
iv) die Verbindung der Formel IX mit (a) einem Benzylierungsmittel in Gegenwart einer Base oder (b) Benzaldehyd in Gegenwart eines Reduktionsmittels und einer Säure behandelt wird, um die Verbindung der Formel I zu bilden.

14. Verbindung der Formel 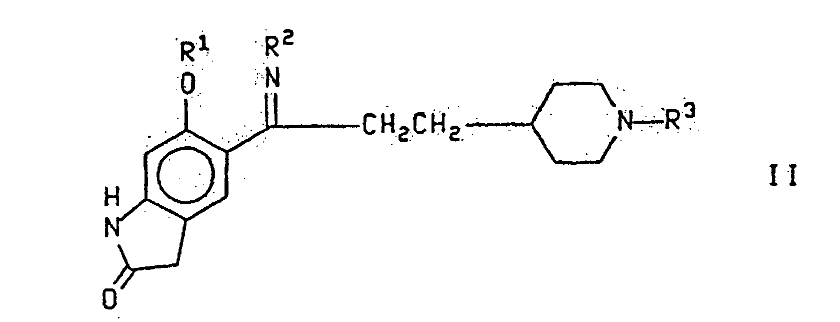 worin R¹ H ist und R² OH oder OR⁴ ist, wobei R⁴ R⁵C(=O), R⁵OC(=O) oder R⁵SO₂ ist, wobei R⁵ (C₁-C₆)-Alkyl, (C₆-C₁₀)-Aryl oder (C₆-C₁₀)-Aryl-(C₁-C₆)-alkyl ist und R³ R⁴ oder Benzyl ist.

15. Verbindung nach Anspruch 14, wobei R¹ Wasserstoff ist und R² Hydroxyl oder OR⁴ ist und R³ und R⁴ wie oben definiert sind.

16. Verbindung nach Anspruch 15, wobei R² OH ist.

17. Verbindung nach Anspruch 15, wobei R² OR⁴ ist und R⁴ wie oben definiert ist.

18. Verbindung nach Anspruch 14, wobei R³ R⁴ ist und R⁴ wie oben definiert ist.

19. Verfahren nach Anspruch 13, Stufe c) iv), wobei das Benzylierungsmittel Benzylbromid ist und die Base Triethanolamin ist.

20. Verfahren nach Anspruch 13, Stufe b), wobei die Base 2,6-Lutidin ist.

## Revendications

1. Procédé de préparation du composé ayant la formule 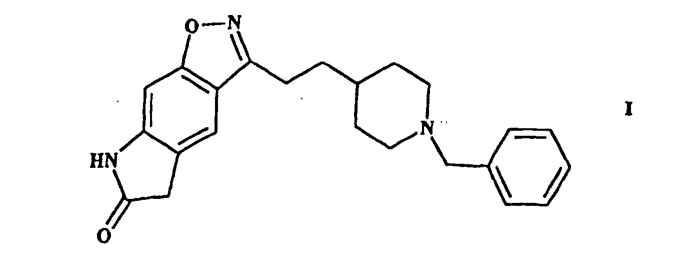 qui comprend :
i) le chauffage d'un composé de formule 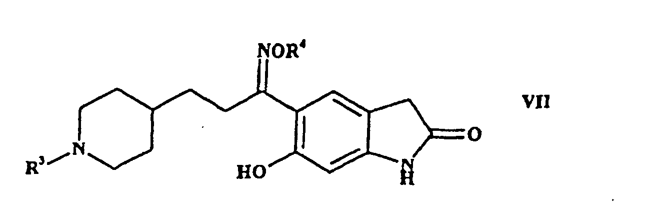 dans laquelle R³ représente R⁴ ou benzyle et R⁴ est sélectionné parmi R⁵-C(=O)-, R⁵SO₂- et R⁵OC(=O)- et R₅ représente alkyle en C₁-C₆, aryle en C₆-C₁₀ ou aryle(alkyle en C₁-C₆) ;
sous l'une des conditions suivantes :
1. à la température de reflux d'un mélange formé avec une base appropriée telle que la pyridine ou la 2,6-lutidine ;
2. à une température d'environ 130°C dans un solvant polaire en présence d'une base ; ou
3. à la température de reflux d'un mélange formé avec du TFH et de la 2,6-lutidine comme base,
étant entendu que, lorsque R³ représente R⁴ dans le produit résultant, ledit produit est
ii) traité en outre avec un acide minéral aqueux à une température comprise dans la gamme allant d'approximativement la température ambiante à approximativement à la température de reflux, suivi par
(iii) le traitement du produit issu de ii) avec
a) un agent de benzylation en présence d'une base, ou
b) du benzaldéhyde en présence d'un agent réducteur et d'un acide.

2. Procédé selon la revendication 1, dans lequel l'acide de l'étape ii) est du HCl 3-12N.

3. Procédé selon la revendication 2, dans lequel ledit acide est du HCl 6N et ladite température est comprise entre environ 80° et environ 90°C.

4. Procédé selon la revendication 1, dans lequel ledit agent de benzylation est un halogénure de benzyle.

5. Procédé selon la revendication 4, dans lequel ledit halogénure de benzyle est du bromure de benzyle.

6. Procédé selon la revendication 1, dans lequel la base de l'étape iii)a) est la triéthanolamine.

7. Procédé selon la revendication 1, dans lequel la base de l'étape i) est la 2,6-lutidine.

8. Procédé selon la revendication 1 étape iii)b), dans lequel ledit agent réducteur est un triacétoxyborohydrure et ledit acide est l'acide acétique.

9. Procédé selon la revendication 1, dans lequel le composé de formule VII est préparé en traitant le composé de formule 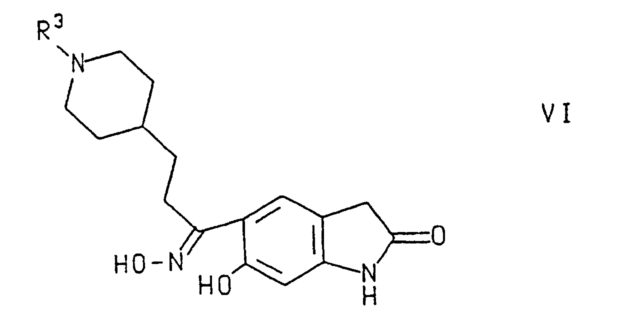 dans laquelle
R³ est tel que défini ci-dessus ;
avec un agent d'acylation, de carbamylation ou de sulfonation en présence d'une base.

10. Procédé selon la revendication 9, dans lequel ledit agent d'acylation est l'anhydride acétique et ladite base est l'acétate de sodium.

11. Procédé selon la revendication 9, dans lequel le composé de formule VI est préparé en traitant le composé de formule 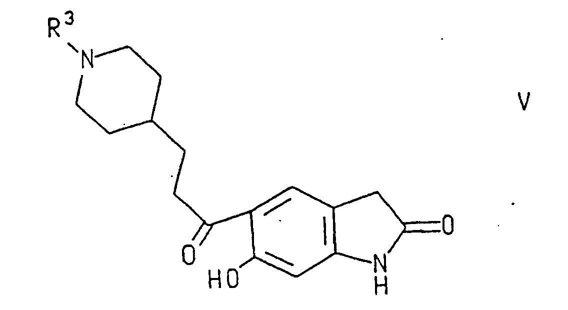 dans laquelle R³ est tel que défini ci-dessus, avec un sel d'hydroxylamine en présence d'une base faible.

12. Procédé selon la revendication 11, dans lequel ledit sel est le chlorhydrate et ladite base faible est l'acétate de sodium.

13. Procédé de préparation d'un composé de formule 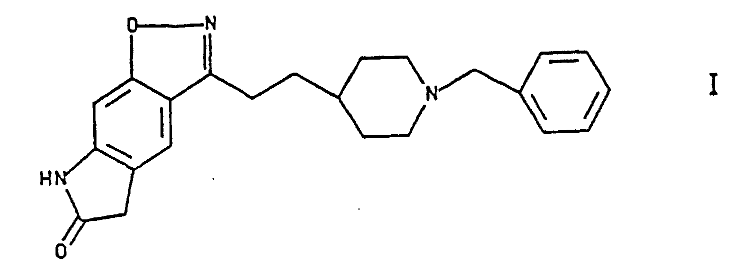 qui comprend les étapes de
(a) traitement d'un composé de formule 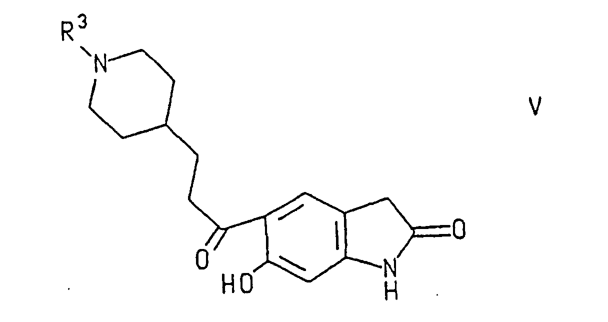 dans laquelle R³ est tel que défini ci-dessus :
avec un sel d'hydroxylamine en présence d'une base faible pour former le composé de formule 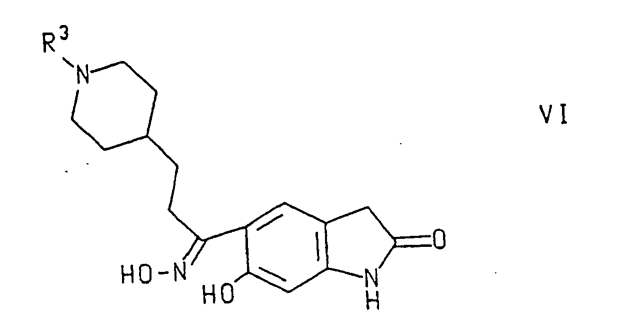 dans laquelle R³ est tel que défini ci-dessus ;
b) le traitement du composé de formule VI avec un agent d'acylation, de carbamylation ou de sulfonation en présence d'une base pour former le composé de formule 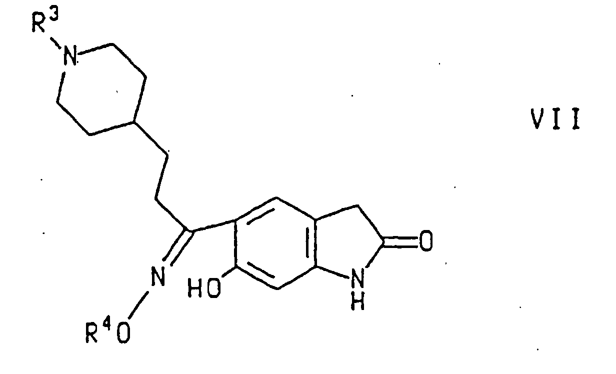 dans laquelle R³ et R⁴ sont tels que définis ci-dessus ; et
c) le traitement du composé de formule VII avec une base à une température élevée selon l'étape i) de la revendication 1 ci-dessus, pour former
i) lorsque R³ représente 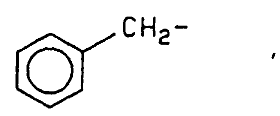 le composé de formule I ; ou
ii) lorsque R³ représente R⁴ un produit de formule 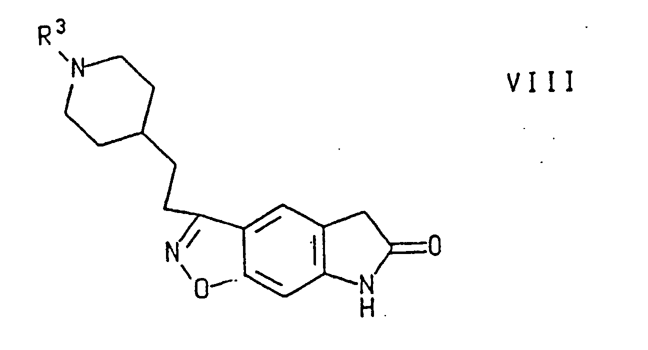 dans laquelle R³ est tel que défini ci-dessus ;
iii) le traitement du composé de formule VIII avec un acide minéral aqueux à une température élevée selon l'étape ii) de la revendication 1 ci-dessus, puis avec une base pour former le composé de formule 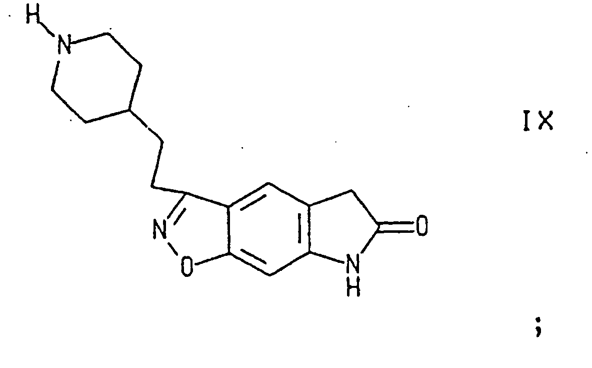 et
iv) le traitement du composé de formule IX avec (a) un agent de benzylation en présence d'une base ou b) le benzaldéhyde en présence d'un agent réducteur et d'un acide pour former le composé de formule I.

14. Composé de formule 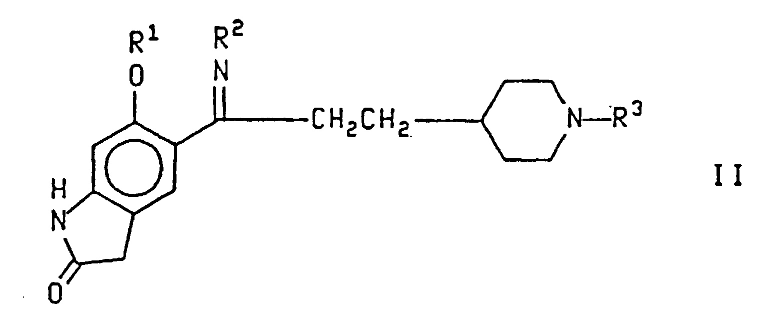 dans laquelle
R¹ représente H et R² représente OH ou OR⁴, où R⁴ représente R⁵C(=O), R⁵OC(=O) ou R⁵SO₂, où R⁵ représente alkyle en C₁-C₆, aryle en C₆₋C₁₀ ou (aryle en C₆-C₁₀)alkyle en C₁-C₆ ; et R³ représente R⁴ ou benzyle.

15. Composé selon la revendication 14, dans lequel R¹ représente l'hydrogène et R² représente hydroxyle ou OR⁴, et R³ et R⁴ sont tels que définis ci-dessus.

16. Composé selon la revendication 15, dans lequel R² représente OH.

17. Composé selon la revendication 15, dans lequel R² représente OR⁴ et R⁴ est tel que défini ci-dessus.

18. Composé selon la revendication 14, dans lequel R³ représente R⁴ et R⁴ est tel que défini ci-dessus

19. Procédé selon la revendication 13 étape c)iv) dans lequel ledit agent de benzylation est le bromure de benzyle et ladite base est la triéthanolamine.

20. Procédé selon la revendication 13 étape b) dans lequel ladite base est la 2,6-lutidine.
